# EUROPEAN PATENT APPLICATION

(11) **EP 2 351 531 A1**
(43) Date of publication of application: **03.08.2011**
(21) Application number: 09827648.8
(22) Date of filing: 02.02.2009
(51) Int. Cl.: A61B 17/34

(54) **BONE MARROW EXTRACTOR**

(30) Priority: 18.11.2008 KR 20080114407
(71) Applicant: Industry-Academic Cooperation Foundation Wonkwang University, Jellabuk-do 570-749 (KR)
(72) Inventor: LEE, Jun, Daejeon-si 305-755 (KR)
(74) Representative: Denjean, Eric
(86) International application number: PCT/KR2009/000503
(87) International publication number: WO 2010/058882

(57) **Abstract**

The present invention relates to a bone marrow extractor. The bone marrow extractor of the present invention has an outer needle rod with a case having an upper portion and a lower portion in communication with each other. The case has a front end with a crushing blade and a rear portion with a stopper groove. An inner needle rod which has a main body having a front end with a crushing blade and a rear portion with a connector is inserted into the outer needle rod. The main body of the inner needle rod has an outer surface from which a stopper lever is protruded. The front end of the main body of the inner needle rod is protruded from the front end of the case of the outer needle rod when the stopper lever is fitted into the stopper groove. The inner needle rod and the outer needle rod cooperate with each other to crush bones through a rotation when the head of a driving means coupled to the connector operates. After bone crushing, only the inner needle rod is removed, and the outer needle rod is kept within the human body to suck and extract bone marrow in a syringe-like manner.

## Description

### Technical Field

The present invention relates to a bone marrow extractor and, more particularly, to a bone marrow extractor which is used to drill a bone to extract bone marrow in the bone.

### Background Art

Generally, bone marrow is extracted using a bone marrow puncture needle to manually puncture the ilium.

This kind of bone marrow puncture needle is configured in such a manner as to insert and fit an inner needle rod into a tubular mantle while allowing the tip of the inner needle rod to project from the mantle.

Then, the bone marrow puncture needle, which is equipped with a handle in the mantle, percutaneously punctures the ilium, and once the tip of the needle reaches the bone marrow, the inner needle rod alone is drawn out and the mantle remains in contact with the bone marrow for subsequent use.

As a method for extracting bone marrow using this bone marrow puncture needle, typically, an aspiration method is employed. The aspiration method is such that the mantle of the bone marrow puncture needle is connected to a syringe to collect bone marrow by virtue of the aspiration force.

In this method, however, since the quantity of bone marrow extracted from one site of a bone is as small as several millimeters, collecting a sufficient quantity of bone marrow requires a number of sites to be punctured. Further, a large amount of peripheral blood is allowed to mix into the extracted bone marrow. Therefore, the method, when used in allogeneic bone marrow transplantation, carries a high risk of causing graft-versus-host disease (GVHD), which requires immunosuppression.

Under these circumstances, a bone marrow perfusion method has been developed as a method for overcoming the above problems. In the bone marrow perfusion method, two bone marrow extracting needles are placed in contact with bone marrow so as to slowly perfuse bone marrow with a perfusion medium such as sterilized physiological saline.

One of the needles is connected to a bone marrow extracting set, and a bone marrow extracting bag of the set is connected to an injection syringe or a tube of a parenteral fluid pump to aspirate bone marrow.

Using a syringe that is filled with a perfusion medium and is connected to the other needle, the perfusion medium is injected slowly into the bone marrow in such a manner as to wash away the bone marrow, and thereby a required quantity of the bone marrow is extracted into the extracting set in a short time. The bone marrow extracted in this way can be used not only for bone marrow transplantation but also for regenerative medicine.

However, the conventionally used bone marrow puncture needle, which is configured to be inserted into a bone manually by holding the handle, is not suitable for application to the bone marrow perfusion method from the viewpoint of the puncture rate and other factors.

Thus, a method that drills and punctures a bone in a short time using a bone marrow extracting needle equipped with an electric drill that is capable of providing powerful rotational power is preferred. For this reason, a bone marrow extracting needle has been proposed that includes an inner needle rod with a drilling edge and a mantle with an angled edge formed at its tip, the mantle being allowed to rotate at a reduced speed with respect to the rotation of the inner needle rod through a speed reduction mechanism (WO 03/015637).

However, this bone marrow extracting needle has the problems that bone scraps may be introduced into a body during drilling of the bone and the bone or body part located adjacent to the bone marrow may be damaged.

### Disclosure

### Technical Problem

The prevent invention has been made in an effort to solve the above-described problems associated with the prior art, and an object of the present invention is to provide a bone marrow extractor including an inner needle rod for drilling and penetrating a bone and an outer needle rod, into which the inner needle rod is inserted and fitted, for drilling the bone and extracting bone marrow, the bone marrow extractor minimizing damage to the bone or body part located adjacent to the bone marrow during extracting of the bone marrow.

### Technical Solution.

In order to accomplish the above object, the present invention relates to a bone marrow extractor. The bone marrow extractor of the present invention has an outer needle rod with a case having an upper portion and a lower portion in communication with each other. The case has a front end with a crushing blade and a rear side with a stopper groove. An inner needle rod which has a main body having a front end with a crushing blade and a rear side with a connector is inserted into the outer needle rod. The main body of the inner needle rod has an outer surface from which a stopper lever is protruded. The front end of the main body of the inner needle rod is protruded from the front end of the case of the outer needle rod when the stopper lever is fitted into the stopper groove. The inner needle rod and the outer needle rod cooperate with each other to crush bones by rotation when the head of a driving means coupled to the connector operates. After bone crushing, only the inner needle rod is removed, and the outer needle rod is kept within the human body to suck and extract bone marrow in a syringe-like manner.

### Advantageous Effects

According to the present invention, the bone marrow extractor including an inner needle rod for drilling and penetrating a bone and an outer needle rod, into which the inner needle rod is inserted and fitted, for drilling the bone and extracting bone marrow minimizes damage to the bone or body part located adjacent to the bone marrow during extracting of the bone marrow.

Moreover, since the crushing blade formed at each of the inner and outer needle rods has a spiral shape, it is possible to reduce load during drilling of the bone.

Furthermore, the inner needle rod is prevented from being inserted too deep by the front end of the outer needle rod having a trapezoidal shape and is drawn out so as to easily aspirate the bone marrow during extracting of the bone marrow.

### Brief Description of Drawings

FIG. 1 is a perspective view showing a bone marrow extractor in accordance with the present invention;
FIG. 2 is a front view showing an outer needle rod of the bone marrow extractor in accordance with the present invention;
FIG. 3 is a front view showing an inner needle rod of the bone marrow extractor in accordance with the present invention;
FIG. 4 is a partial cross-sectional view showing an assembled state of the bone marrow extractor in accordance with the present invention; and
FIGS. 5 to 8 are front views showing the use state of the bone marrow extractor in accordance with the present invention.

### Best Mode for Invention

Hereinafter, exemplary embodiments of the present invention will be described in detail below with reference to the accompanying drawings such that those skilled in the art to which the present invention pertains can easily practice the present invention.

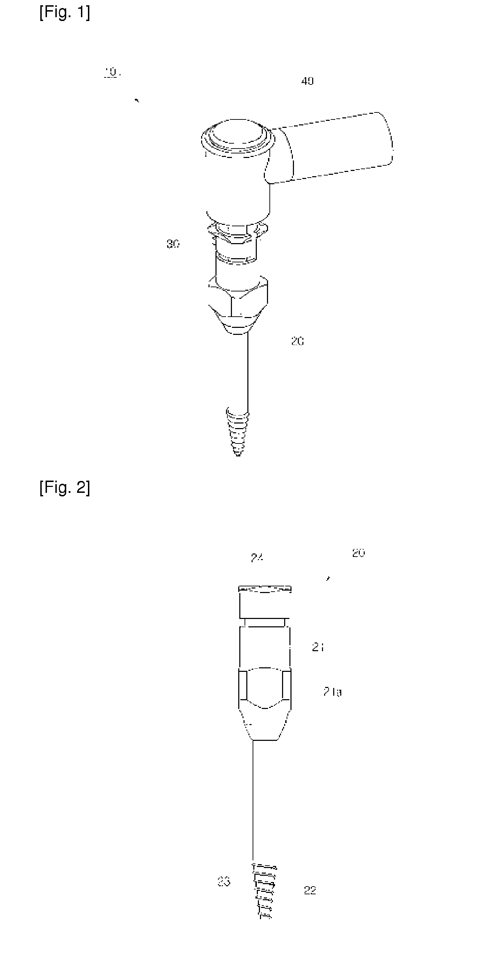

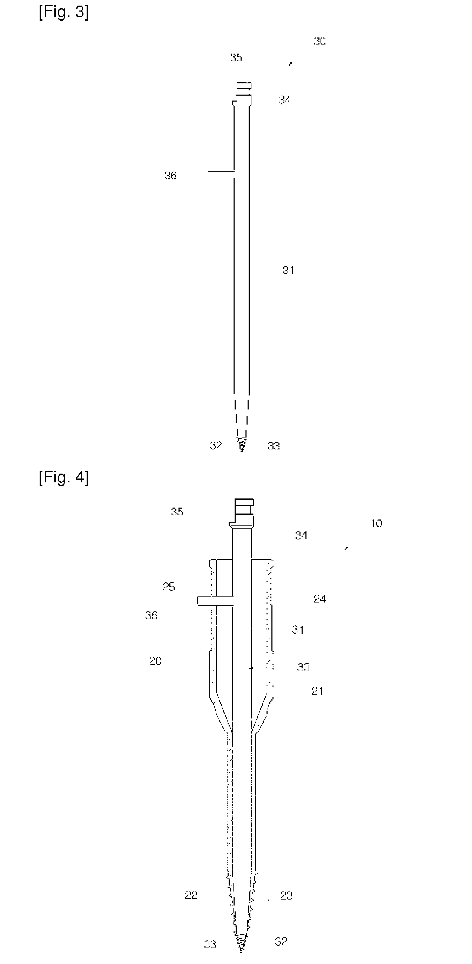

FIG. 1 is a perspective view showing a bone marrow extractor in accordance with the present invention, FIG. 2 is a front view showing an outer needle rod of the bone marrow extractor in accordance with the present invention, FIG. 3 is a front view showing an inner needle rod of the bone marrow extractor in accordance with the present invention, and FIG. 4 is a partial cross-sectional view showing an assembled state of the bone marrow extractor in accordance with the present invention.

A bone marrow extractor 10 of the present invention includes a hollow outer needle rod 20, an inner needle rod 30 inserted and fitted into the outer needle rod 20 to drill a bone together with the outer needle rod 20 by a rotation operation, and a head 40 of a driving means (not shown) such as an engine or motor which applies a rotational force to the inner needle rod 30.

The outer needle rod 20 includes a case 21, a crushing blade 23 formed at a front end 22 of the case 21, and a stopper groove 25 formed at a rear side 24 of the case 21.

The case 21 has a hollow cylindrical shape in which the front end 22 has a diameter smaller than that of the rear side 24 and includes a polygonal rotating member 21 a formed in a middle portion thereof so as to rotate the case 21 using a wrench or spanner.

Here, the front end 22 has a trapezoidal shape to gently drill the bone 50, and the crushing blade 23 formed at the front end 22 has a spiral shape to smoothly discharge bone scraps, generated during drilling of the bone 50, to the outside.

Moreover, the stopper groove 25 formed at the rear side 24 has an "L" shape to allow the upper end thereof to be connected to the outside and has a width greater than that of a stopper lever, which will be described later, of the inner needle rod 30.

That is, the stopper groove 25 includes a vertical member 25a penetrating vertically and a horizontal member 25b connected integrally to the vertical member 25a so as to form an "L" shape having a width greater than that of the stopper lever.

The inner needle rod 30 inserted and fitted into the outer needle rod 20 includes a main body 31 having a predetermined length, a crushing blade 33 formed at a front end 32 of the main body 31, a connection member 35 formed at a rear end 34 of the main body 31, and the stopper lever 36 projecting outwardly from a middle portion of an outer circumferential surface of the main body 31, which are integrally formed with each other.

Here, the main body 31 is formed to have a cylindrical shape having a predetermined length.

Moreover, the front end 32 has a sharp tip to gently drill the bone 50, and the crushing blade 33 formed at the front end 32 of the main body 31 has a spiral shape to smoothly discharge bone scraps, generated during drilling of the bone 50, to the outside.

The connection member 35 formed at the rear end 34 has an axial shape to be connected with the head 40, and the stopper lever 36 formed on the outer circumferential surface of the main body 31 projects a predetermined distance outwardly.

The head 40 of the driving means such as a forward/reverse motor or engine is connected to the connection member 35 of the inner needle rod 30 to apply a rotational force to the inner needle rod 30.

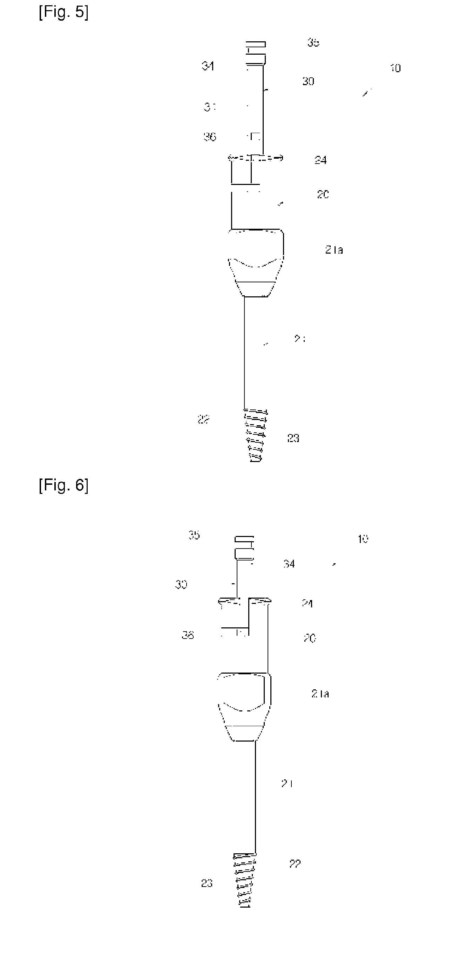

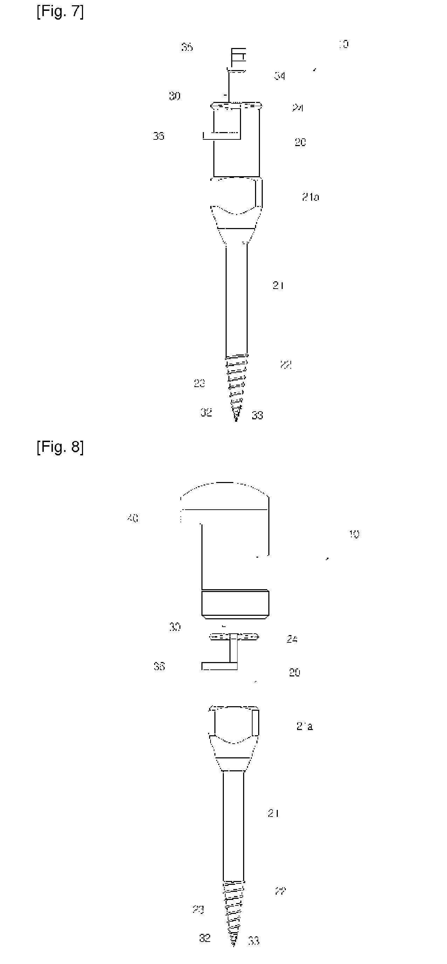

A preferred embodiment of the bone marrow extractor 10 having the above-described configuration in accordance with the present invention will be described with reference to FIGS. 5 to 8.

FIGS. 5 to 8 are front views showing the use state of the bone marrow extractor in accordance with the present invention.

First, the outer needle rod 20 including the hollow case 21, the crushing blade 23 formed at the front end 22 of the case 21, and the stopper groove 25 formed at the rear side 24 of the case 21, which are integrally formed with each other, is provided.

Here, the crushing blade 23 has a spiral shape, and the stopper groove 25 has an "L" shape.

Then, the inner needle rod 30 including the main body 31 having a predetermined length, the crushing blade 33 formed at the front end 32 of the main body 31, the connection member 35 formed at the rear end 34 of the main body 31, and the stopper lever 36 projecting outwardly from the outer circumferential surface of the main body 31, which are integrally formed with each other, is inserted and fitted into the outer needle rod 20.

Here, when the stopper lever 36 is inserted into the stopper groove 25, the front end 32 of the main body 31 projects through the front end 22 of the case 21.

That is, since the spiral crushing blade 33 of the inner needle rod 30 and the spiral crushing blade 23 of the outer needle rod 20 are continuously formed so as to gently drill the bone 50 and, at the same time, to smoothly discharge bone scraps during the rotation operation.

Next, the connection member 35 of the inner needle rod 30 is connected to the head 40 of the driving means which applies a rotational force to the inner needle rod 30, thus, completing the assembling of the bone marrow extractor 10 to be used.

When the head 40 of the driving means is operated to extract bone marrow, the inner needle rod 30 and the outer needle rod 20 rotate in the same direction to drill the bone 50.

That is, the inner needle rod 30 transmits the rotational force from the head 40 of the driving means to the outer needle rod 20, thus drilling the bone 50 together with the outer needle rod 20.

When the drilling of the bone 50 by the rotation operation of the inner needle rod 30 and the outer needle rod 20 is completed, the inner needle rod 30 inserted into the outer needle rod 20 is drawn out and the outer needle rod 20 remains in contact with the bone marrow so as to aspirate the bone marrow through the opening formed at the bottom of the outer needle rod 20.

### Industrial Applicability

As described above, it is possible to minimize damage to the bone or body part located adjacent to the bone marrow during extracting of bone marrow using the bone marrow extractor 10 having the above-described configuration in accordance with the present invention.

The invention has been described in detail with reference to preferred embodiments thereof. However, it will be appreciated by those skilled in the art that changes may be made in these embodiments without departing from the principles and spirit of the invention, the scope of which is defined in the appended claims and their equivalents.

## Claims

1. A bone marrow extractor comprising:
an outer needle rod including a case having an upper portion and a lower portion in communication with each other, the case having a front end with a crushing blade and a rear side with a stopper groove; and
an inner needle rod including a main body having a front end with a crushing blade and a rear side with a connector and inserted into the outer needle rod,
wherein the inner needle rod comprises a stopper lever formed to project from the outer circumference of the main body and connected to the stopper groove to rotate with each other.

2. The bone marrow extractor of claim 1, wherein the front end of the main body of the inner needle rod is protruded from the front end of the case of the outer needle rod to form a wedge shape when the stopper lever is fitted into the stopper groove.

3. The bone marrow extractor of claim 1, wherein the crushing blade at the front end of the outer needle rod and the crushing blade at the front end of the inner needle rod have a spiral shape, respectively.

4. The bone marrow extractor of claim 1, wherein the stopper groove of the outer needle rod, into which the stopper lever is inserted, has an "L" shape such that the inner needle freely rotates with respect to the outer needle at a predetermined angle.

5. The bone marrow extractor of claim 1, wherein the case has a hollow cylindrical shape, in which the front end has a diameter smaller than that of the rear side, and comprises a polygonal rotating member formed in a middle portion thereof so as to rotate the case using a wrench or spanner.
